Europäisches Patentamt

European Patent Office

Office européen des brevets

(19)

(11) Veröffentlichungsnummer : **0 230 881 B1**

(12) **EUROPÄISCHE PATENTSCHRIFT**

(45) Veröffentlichungstag der Patentschrift :
**15.05.91 Patentblatt 91/20**

(51) Int. Cl.⁵ : **A61K 31/495, A61K 9/20,**
**A61K 47/02, A61K 47/30,**
**A61K 47/38**

(21) Anmeldenummer : **87100173.1**

(22) Anmeldetag : **09.01.87**

(54) **Pharmazeutische Zubereitungen von Ciprofloxacin.**

Die Akte enthält technische Angaben, die nach dem Eingang der Anmeldung eingereicht wurden und die nicht in dieser Patentschrift enthalten sind.

(30) Priorität : **21.01.86 DE 3601566**

(43) Veröffentlichungstag der Anmeldung :
**05.08.87 Patentblatt 87/32**

(45) Bekanntmachung des Hinweises auf die Patenterteilung :
**15.05.91 Patentblatt 91/20**

(84) Benannte Vertragsstaaten :
**AT BE CH DE ES FR GB GR IT LI NL SE**

(56) Entgegenhaltungen :
**EP-A- 0 049 355**
**EP-A- 0 164 619**
**GB-A- 2 160 519**
**US-A- 4 017 622**
**US-A- 4 620 007**

(73) Patentinhaber : **BAYER AG**
**W-5090 Leverkusen 1 Bayerwerk (DE)**

(72) Erfinder : **Streuff, Bernd, Dr.**
**Asterweg 23**
**W-5632 Wermelskirchen 2 (DE)**
Erfinder : **Luchtenberg, Helmut, Dr.**
**Windmühlenstrasse 11a**
**W-5216 Niederkassel 6 (DE)**

**Beschreibung**

Die Erfindung betrifft pharmazeutische Zubereitungen der 1-Cyclopropyl-6-fluor-1,4-dihydro-4-oxo-7-(1-piperazinyl)-chinolin-3-carbonsäure, im folgenden auch Ciprofloxacin genannt, Verfahren zu ihrer Herstellung sowie Kapseln und Tabletten, die solche Zubereitungen enthalten. .

Die Verwendung von 1-Cyclopropyl-6-fluor-1,4-dihydro-4-oxo-7-(1-piperazinyl)-chinolin-3-carbonsäure und ihre physiologisch verträglichen Derivate ist aus der europäischen Patentanmeldung 0 049 355 und der deutschen Patentanmeldung 3 142 854 bekannt. Zur Injektion und Infusion geeignete milchsaure Lösungen des Ciprofloxacins werden in der deutschen Patentanmeldung 3 333 719 beschrieben.

Aus der EP-A-0 049 355 ist bekannt, daß es sich bei Ciprofloxacin um einen hochwirksamen antibakteriellen Wirkstoff handelt, der auch oral verabreicht werden kann. In dieser Veröffentlichung werden denkbare Tablettenzusammensetzungen allgemein beschrieben, derartige Zusammensetzungen sind jedoch von ihren Eigenschaften her allgemein nicht zufriedenstellend. Aus der EP-A-0 64 619 ist eine Tablettenzusammensetzung bekannt geworden, die enthält. Dieser Wirkstoff ist dem Ciprofloxacin jedoch deutlich unterlegen.

Die Erfindung betrifft oral applizierbare pharmazeutische Zubereitungen, die aus

72,4 bis 78,8 Gew.-% 1-Cyclopropyl-6-fluor-1,4-dihydro-4-oxo-7-(1-piperazinyl)-chinolin-3-carbonsäure als HCl-Salz-Monohydrat ;

7,0 bis 9,0 Gew.-% mikrokristalliner Cellulose (Trocken binde mittel) ;

9,0 bis 12,0 Gew.-% Maisstärke (Zerfallhilfsmittel) ;

4,0 bis 5,0 Gew.-% quervernetztem Polyvinylpyrrolidon ;

0,6 bis 0,8 Gew.-% kolloidalem Siliciumdioxid (Fließmittel) und

0,6 bis 0,8 Gew.-% Schmiermittel bestehen.

Die erfindungsgemäßen pharmazeutischen Zubereitungen vereinen hohe biologische Verfügbarkeit mit ausgezeichneter Haltbarkeit.

Als Trockenbindemittel wird bevorzugt eine hochgereinigte, mikrokristalline Cellulose vom Molgewicht 30 000 bis 50 000 mit einer Teilchengröße von 10 bis 50 µm und einem Wassergehalt von 4 bis 6 Gew.-% verwendet.

Als Zerfallhilfsmittel kommen einer gebräuchliche Stärkearten, insbesondere jedoch Maisstärke zur Anwendung, zum anderen aber auch Cellulosederivate und/oder quervernetzte Polyvinylpyrrolidone.

Für diesen Zweck gebräuchliche Cellulosederivate sind z.B. Natriumcarboxymethylcellulose.

Quervernetztes PVP ist im Handel erhältlich z.B. unter den Handelsnamen Kollidon® Cl (BASF AG, Ludwigshafen (D) oder Plasdone® XL (General Aniline & Film Corp., New York (USA)).

Als Fließmittel kommen pulverförmige, oft auch als Pudergrundstoffe oder als Pudergrundlagen verwendete Stoffe in Betracht, welche die Eigenschaften besitzen, anderen pulverförmigen Stoffen, die eine gewisse Adhärenz aufweisen, ein besseres Fließ- und Schüttvermögen zu vermitteln. Es eignen sich beispielsweise Aerosil®, ein hochreines röntgenamorphes Siliciumdioxid (> 99,8% $SiO_2$), Aerosil® 972, ein reines Siliciumdioxid, das durch chemisch veränderte Methylgruppen hydrophobe Eigenschaften aufweist, und NAL®, NAL® RS, ein aus Reisstärke hergestelltes pulverförmiges Produkt (S. auf H.P. Fiedler, Lexikon der Hilfsstoffe für Pharmazie. Kosmetik und angrenzende Gebiete, Editio Captor KG, Aulendorf i. Württ. (D)).

Schmiermittel sind beispielsweise Talkum, Calcium- und Magnesiumstearat sowie feste Polyethylenglykole. Bevorzugt ist Magnesiumstearat.

Weiterer Gegenstand der Erfindung sind Verfahren zur Herstellung der erfindungsgemäßen Wirkstoffzubereitungen.

Dazu wird der Wirkstoff Ciprofloxacin in einer Menge von 72,4 bis 78,8 Gew.-%, bezogen auf die Gesamtmenge der Zubereitung mit 7,0 bis 9,0 Gew.-% eines Trockenbindemittels auf der Basis von Cellulose, 9,0 bis 12,0 Gew.-% eines Zerfallshilfsmittels auf der Basis von Stärke und 0,6 bis 0,8 Gew.-% eines Fließverbesserers vermischt, die Mischung mit Wasser bis zum Entstehen einer granulierfähigen Mischung angefeuchtet, granuliert, getrocknet, gegebenenfalls gesiebt, und anschließend mit 4,0 bis 5,0 Gew.-% eines Zerfallshilfsmittels auf der Basis von Cellulosederivaten und/oder quervernetzten Polyvinylpyrrolidonen und 0,6 bis 0,8 Gew.-% eines Schmiermittels vermischt und zu Tabletten verpreßt oder in Kapseln abgefüllt.

Eine Variante des oben geschilderten Verfahrens besteht darin, daß man die Wirkstoffmischung in einem Wirbelschichtgranulator durch kontinuierliches Sprühen von Wasser ofer wäßrigen Bindemittellösungen und gleichzeitige Zufuhr von Warmluft granuliert, das erhaltene Granulat siebt und gegebenenfalls zu Tabletten verpreßt.

In einer weiteren Variante wird der Wirkstoff Ciprofloxacin mit dem Trockenbindemittel auf der Basis von Cellulose, gegebenenfalls in Gegenwart eines Zerfallshilfsmittels auf der Basis von Stärke und mit dem weiteren Zerfallhilfsmittel auf der Basis von Cellulosederivaten und/oder quervernetztem Polyvinylpyrrolidon granuliert und das Granulat zersiebt und gegebenenfalls mit den restlichen Zuschlagstoffen vermischt und in Tabletten verpreßt oder in Kapseln abgefüllt.

Zweckmäßigerweise werden durch das Aussieben Granulate mit einem Querschnitt von 0,8 bis 2 mm für die weitere Verarbeitung zu Tabletten oder Kapseln bereit gestellt.

Bevorzugt kann man auch so verfahren, daß die Wirkstoffe mit Maisstärke, Avicel® und Aerosil® vermischt werden und diese Mischungen nach dem Granulieren mit quervernetztem Polyvinylpyrrolidon und Magnesium-stearat vereinigt und anschließend zu Tabletten verpreßt werden.

Die erfindungsgemäßen Zubereitungen zeigen bei geringer Toxizität ein breites antibakterielles Spektrum gegen gram-positive und gram-negative Keime, insbesondere gegen Enterobakteriaceen, vor allem auch gegen solche, die resistent sind gegen verschiedene Antibiotika, wie z.B. Penicilline, Cephalosphorine, Aminoglykoside, Sulfonamide, Tetracycline.

Diese wertvollen Eigenschaften ermöglichen ihre Verwendung als chemotherapeutische Wirkstoffe in der Medizin.

Die erfindungsgemäßen Zubereitungen sind gegen ein sehr breites Spektrum von Mikroorganismen wirksam. Mit ihrer Hilfe können gram-negative und gram-positive Bakterien und bakterienähnliche Mikroorganismen bekämpft sowie die durch diese Erreger hervorgerufenen Erkrankungen verhindert, gebessert und/oder geheilt werden.

Besonders wirksam sind die erfindugsgemäßen Zubereitungen gegen Bakterien und bakterieähnliche Mikroorganismen. Sie sind daher besonders gut zur Prophylaxe und Chemotherapie von lokalen und systemischen Infektionen in der Human- und Tiermedizin geeignet, die durch diese Erreger hervorgerufen werden.

Beispielsweise können lokale und/oder systemische Erkrankungen behandelt und/oder verhindert werden, die durch die folgenden Erreger oder durch Mischungen der folgenden Erreger verursacht werden :

Micrococcaceae, wie Staphylokokken, z.B. Staph. aureus, Staph. Epidermidis, (Staph. = Staphyloccoccos) ; Lactobacteriaceae, wie Streptokokken, z.B. Streptococcus pyogenes, α- bzw. β-hämolysierende Streptokokken, nicht-γ-hämolysierende Streptokokken, Enterokokken und Diplocuccus pneumoniae (pneumokokken) Enterobacteriaceae, wie Escherichiae-Bakterien der Escheridrion-Gruppe, z.B. Escherichia coli, Enterobacter-Bakterien, z.B. E. aerogenes, E. Cloacae (E. = Enterobacter), Klebsiella-Bakterien, z.B. K. pneumoniae (K. = Klebsiella), Serratia, z.B. Serratia marcescens, Proteae-Bakterien der Proteus-Gruppe ; Proteus, z.B. Pr. vulgaris, Pr. morganii, Pr. retgeri, Pr. mirabilis (Pr. = Proteus) ; Pseudomonadaceae, wie Pseudomonas-Bakterien, z.B. Ps. aeruginosa (Ps. = Pseudomonas) ; Bacteroidaceae, wie Bacteriodes-Bakterien, z.B. Bacteroides fragilis ; Mykoplasmen, z.B. Mykoplasma pneumonia, außerdem Mykobakterien, z.B. Mykobacterium tuberculosis, Mycobacterium leprae und atypische Mikrobakterien.

Die obige Aufzählung von Erregern ist lediglich beispielshaft und keinesweg beschränkt aufzufassen. Als Krankheiten, die durch die erfindungsgemäßen Zubereitungen verhindert, gebessert und/oder geheilt werden können, seien beispielsweise genannt :

Otitis ; Pharyngitis ; Pneumonie ; Peritonitis ; Pyelonephritis ; Cystitis ; Endocarditis ; Systeminfektionen ; Bronchitis ; Arthritis ; lokale Infektionen ; septische Erkrankungen.

Zur vorliegenden Erfindung gehören auch pharmazeutische Zubereitungen in Dosierunggseinheiten. Dies bedeutet, daß die Zubereitungen in Form einzelner Teile, z.B. Tabletten, Dragees, Kapseln und Pillen vorliegen, deren Wirkstoffgehalt einem Bruchteil oder einem Vielfachen einer Einzeldosis entsprechen. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Die Tabletten, Dragees, Kapseln, Pillen und Granulate können mit den üblichen, gegebenenfalls Opakisierungsmitteln enthaltenden, Überzügen und Hüllen versehen sein und auch so zusammengesetzt sein, daß sie den oder die Wirkstoffe nur oder bevorzugt in einem bestimmten Teil des Intestinaltraktes gegebenenfalls verzögert abgeben, wobei als Einbettungsmassen, z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Die erfindugsgemäßen Formulierungsformen können auch Färbemittel, Konservierungsstoffe sowie geruchs- und geschmacksverbessernde Zusätze, z.B. Pfefferminzöl und Eukalyptusöl und Süßmittel, z.B. Saccharin, enthalten.

## Beispiel

| | |
|---|---|
| Ciprofloxacin Hydrochlorid-Monohydrid (≙500 mg Betain) | 583,0 mg |
| mikrokristalline Cellulose (AVICEL) | 55,0 mg |
| Maisstärke, feucht | 72,0 mg |
| quervernetztes PVP (Polyplasdone XL) | 30,0 mg |
| Siliciumdioxid | 5,0 mg |
| Magnesiumstearat | 5,0 mg |
| Tablettengewicht | 750,0 mg |

Lackhülle:

| | |
|---|---|
| Hydroxypropylmethylcellulose 15 cp | 6,0 mg |
| PEG 4000 | 2,0 mg |
| Titandioxid | 2,0 mg |
| Tablette lack. | 760,0 mg |

## Ansprüche

**Patentansprüche für die Vertragsstaaten AT, BE, CH, DE, FR, GB, IT, LI, NL, SE**

1. Pharmazeutische Zubereitungen, enthaltend 72,4 bis 78,8 Gew.-% 1-Cyclopropyl-6-fluor-1,4-dihydro-4-oxo-7-(1-piperazinyl)-chinolin-3-carbonsäure als HCl-Salz-Monohydrat,
7,0 bis 9,0 Gew.-% eines Trockenbindemittels auf der Basis von Cellulose,
9,0 bis 12,0 Gew.-% eines Zerfallhilfsmittels auf der Basis von Stärke,
4,0 bis 5,0 Gew.-% eines Zerfallhilfsmittels auf der Basis von Cellulosederivaten und/oder quervernetzten Polyvinylpyrrolidonen,
0,6 bis 0,8 Gew.-% eines Fließverbesserers und
0,6 bis 0,8 Gew.-% eines Schmiermittels.
2. Pharmazeutische Zubereitungen nach Anspruch 1, dadurch gekennzeichnet, daß sie
72,4 bis 78,8 Gew.-% 1-Cyclopropyl-6-fluor-1,4-dihydro-4-oxo-7-(1-piperazinyl)-chinolin-3-carbonsäure als HCl-Salz-Monohydrat,
7,0 bis 9,0 Gew.-% mikrokristalline Cellulose,
9,0 bis 12,0 Gew.-% Maisstärke,
4,0 bis 5,0 Gew.-% quervernetztes Polyvinylpyrrolidon,
0,6 bis 0,8 Gew.-% kolloidales Siliciumdioxid,

0,6 bis 0,8 Gew.-% Magnesiumstearat enthalten.

3. Verfahren zur Herstellung von pharmazeutischen Zubereitungen nach Anspruch 1, dadurch gekennzeichnet, daß man

72,4 bis 78,8 Gew.-% 1-Cyclopropyl-6-fluor-1,4-dihydro-4-oxo-7-(1-piperazinyl)-chinolin-3-carbonsäure-Hydroclorid Monohydrat mit

0,6 bis 0,8 Gew.-% Magnesiumstearat, dadurch gekennzeichnet, daß man 72,4 bis 78,8 Gew.-% 1-Cyclopropyl-6-fluor-1,4-dihydro-4-oxo-7-(1-piperazinyl)-chinolin-3-carbonsäure-Hydrochlorid Monoshydrat mit 7,0 bis 9,0 Gew.-% mikrokristalliner Cellulose, 9,0 bis 12,0 Gew.-% Maisstärke und 0,6 bis 0,8 Gew.-% kolloidalem Siliciumdioxid mischt, zu der Mischung Wasser bis zum Entstehen einer granulierfähigen Masse gibt, granuliert, trocknet, gegebenenfalls siebt, wobei die Porenweiten 0,8 bis 2 mm betragen, und anschließend mit 4,0 bis 5,0 Gew.-% eines Zerfallhilfsmittels auf der basis von quervernetztem Polyvinylpyrrolidon und 0,6 bis 0,8 Gew.-% eines Schmiermittels mischt.

3. Verfahren zur Herstellung von Kapseln oder Tabletten, die den Wirkstoff 1-Cyclopropyl-6-fluor-1,4-dihydro-4-oxo-7-(1-piperazinyl)-chinolin-3-carbonsäure enthalten, dadurch gekennzeichnet, daß man nach Ansprüchen 1 und 2 erhalten Zubereitungen in Kapseln abfüllt oder zu Tabletten verpreßt.

## Patentansprüche für die Vertragsstaaten ES, GR

1. Verfahren zur Herstellung von pharmazeutischen Zubereitungen, enthaltend 72,4 bis 78,8 Gew.-% 1-Cyclopropyl-6-fluor-1,4-dihydro-4-oxo-7-(1-piperazinyl)-chinolin-3-carbonsäure als HCl-Salz-Monohydrat,

7,0 bis 9,0 Gew.-% eines Trockenbindemittels auf der Basis von Cellulose,

9,0 bis 12,0 Gew.-% eines Zerfallhilfsmittels auf der Basis von Stärke,

4,0 bis 5,0 Gew.-% eines Zerfallhilfsmittels auf der Basis von Cellulosederivaten und/oder quervernetzten Polyvinylpyrrolidonen,

0,6 bis 0,8 Gew.-% eines Fließverbesserers und

0,6 bis 0,8 Gew.-% eines Schmiermittels, dadurch gekennzeichnet, daß man 72,4 bis 78,8 Gew.-% 1-Cyclopropyl-6-fluor-1,4-dihydro-4-oxo-7-(1-piperazinyl)-chinolin-3-carbonsäure-Hydrochlorid Monohydrat mit 7,0 bis 9,0 Gew.-% eines Trockenbindemittels auf der Basis von Cellulose, 9,0 bis 12,0 Gew.-% eines Zerfallhilfsmittels auf der Basis von Stärke und 0,6 bis 0,8 Gew.-% eines Fließverbesserers mischt, zu der Mischung Wasser bis zum Entstehen einer granulierfähigen Masse gibt, granuliert, trocknet, gegebenenfalls siebt, wobei die Porenweiten 0,8 bis 2 mm betragen, und anschließend mit 4,0 bis 5,0 Gew.-% eines Zerfallhilfsmittels auf der Basis von Cellulosederivaten und/oder quervernetzten Polyvinylpyrrolidonen und 0,6 bis 0,8 Gew.-% eines Schmiermittels mischt.

2. Verfahren zur Herstellung von pharmazeutischen Zubereitungen nach Anspruch 1 enthaltend

72,4 bis 78,8 Gew.-% 1-Cyclopropyl-6-fluor-1,4-dihydro-4-oxo-7-(1-piperazinyl)-chinolin-3-carbonsäure als HCl-Salz-Monohydrat,

7,0 bis 9,0 Gew.-% mikrokristalline Cellulose,

9,0 bis 12,0 Gew.-% Maisstärke,

4,0 bis 5,0 Gew.-% quervernetztes Polyvinylpyrrolidon,

0,6 bis 0,8 Gew.-% kolloidales Siliciumdioxid,

7,0 bis 9,0 Gew.-% eines Trockenbindemittels auf der Basis von Cellulose,

9,0 bis 12,0 Gew.-% eines Zerfallhilfsmittels auf der Basis von Stärke und

0,6 bis 0,8 Gew.-% eines Fließverbesserers mischt, zu der Mischung Wasser bis zum Entstehen einer granulierfähigen Masse gibt, granuliert, trocknet, gegebenenfalls siebt, wobei die Porenweiten 0,8 bis 2 mm betragen, und anschließend mit 4,0 bis 5,0 Gew.-% eines Zerfallhilfsmittels auf der Basis von Cellulosederivaten und/oder quervernetzten Polyvinylpyrrolidonen und

0,6 bis 0,8 Gew.-% eines Schmiermittels mischt.

4. Verfahren zur Herstellung von Kapseln oder Tabletten, die den Wirkstoff 1-cyclopropyl-6-fluor-1,4-dihydro-4-oxo-7-(1-piperazinyl)-chinolin-3-carbonsäure enthalten, dadurch gekennzeichnet, daß man Zubereitungen nach Ansprüchen 1 und 2 in Kapseln abfüllt oder zu Tabletten verpreßt.

5. Kapseln, enthaltend pharmazeutische Zubereitungen gemäß Ansprüchen 1 und 2.

6. Tabletten, bestehend aus pharmazeutischen Zubereitungen gemäß Ansprüchen 1 und 2.

**Claims**

**Claims for the Contracting States : AT, BE, CH, DE, FR, GB, IT, LI, NL, SE**

1. Pharmaceutical formulations containing 72.4 to 78.8% by weight of 1-cyclopropyl-6-fluoro-1,4-dihydro-4-oxo-7-(1-piperazinyl)-quinoline-3-carboxylic acid as the HCL salt monohydrate, 7.0 to 9.0% by weight of a dry binder based on cellulose, 9.0 to 12.0% by weight of a disintegration auxiliary based on starch, 4.0 to 5.0% by weight of a disintegration auxiliary based on cellulose derivatives and/or crosslinked polyvinylpyrrolidones, 0.6 to 0.8% by weight of a flow-improving agent and 0.6 to 0.8% by weight of a lubricant.

2. Pharmaceutical formulations according to Claim 1, characterized in that they contain 72.4 to 78.8% by weight of 1-cyclopropyl-6-fluoro-1,4-dihydro-4-oxo-7-(1-piperazinyl)-quinoline-3-carboxylic acid as the HCl salt monohydrate, 7.0 to 9.0% by weight of microcrystalline cellulose, 9.0 to 12.0% by weight of maize starch, 4.0 to 5.0% by weight of crosslinked polyvinylpyrrolidone, 0.6 to 0.8% by weight of colloidal silicon dioxide and 0.6 to 0.8% by weight of magnesium stearate.

3. Process for the preparation of pharmaceutical formulations according to Claim 1, characterized in that 72.4 to 7 8.8% by weight of 1-cyclopropyl-6-fluoro-1,4-dihydro-4-oxo-7-(1-piperazinyl)-quinoline-3-carboxylic acid hydrochloride monohydrate are mixed with 7.0 to 9.0% by weight of a dry binder based on cellulose, 9.0 to 12.0% by weight of a disintegration auxiliary based on starch and 0.6 to 0.8% by weight of a flow-improving agent, water is added to the mixture until a composition which can be granulated is formed, the mixture is granulated and the granules are dried and, if appropriate, sieved the pore widths being 0.8 to 2 mm, and then mixed with 4.0 to 5.0% by weight of a disintegration auxiliary based on cellulose derivatives and/or crosslinked polyvinylpyrrolidones and 0.6 to 0.8% by weight of a lubricant.

4. Process for the preparation of capsules or tablets containing the active compound 1-cyclopropyl-6-fluoro-1,4-dihydro-4-oxo-7-(1-piperazinyl)-quinoline-3-carboxylic acid, characterized in that formulations according to Claims 1 and 2 are introduced into capsules or pressed to tablets.

5. Capsules containing pharmaceutical formulations according to Claims 1 and 2.

6. Tablets consisting of pharmaceutical formulations according to Claims 1 and 2.

**Claims for the Contrating States : ES, GR**

1. Process for the preparation of pharmaceutical formulations containing 72.4 to 78.8% by weight of 1-cyclopropyl-6-fluoro-1,4-dihydro-4-oxo-7-(1-piperazinyl)-quinoline-3-carboxylic acid as the HCL salt monohydrate, 7.0 to 9.0% by weight of a dry binder based on cellulose, 9.0 to 12.0% by weight of a disintegration auxiliary based on starch, 4.0 to 5.0% by weight of a disintegration auxiliary based on cellulose derivatives and/or crosslinked polyvinylpyrrolidones, 0.6 to 0.8% by weight of a flow-improving agent and 0.6 to 0.8% by weight of a lubricant, characterized in that 72.4 to 78.8% by weight of 1-cyclopropyl-6-fluoro-1,4-dihydro-4-oxo-7-(1-piperazinyl)-quinoline-3-carboxylic acid hydrochloride monohydrate are mixed with 7.0 to 9.0% by weight of a dry binder based on cellulose, 9.0 to 12.0% by weight of a disintegration auxiliary based on starch and 0.6 to 0.8% by weight of a flow-improving agent, water is added to the mixture until a composition which can be granulated is formed, the mixture is granulated and the granules are dried and, if appropriate, sieved the pore widths being 0.8 to 2 mm, and then mixed with 4.0 to 5.0% by weight of a disintegration auxiliary based on cellulose derivatives and/or crosslinked polyvinylpyrrolidones and 0.6 to 0.8% by weight of a lubricant.

2. Process for the preparation of pharmaceutical formulations according to Claim 1, containing 72.4 to 78.8% by weight of 1-cyclopropyl-6-fluoro-1,4-dihydro-4-oxo-7-(1-piperazinyl)-quinoline-3-carboxylic acid as the HCl salt monohydrate, 7.0 to 9.0% by weight of microcrystalline cellulose, 9.0 to 12.0% by weight of maize starch, 4.0 to 5.0% by weight of crosslinked polyvinylpyrrolidone, 0.6 to 0.8% by weight of colloidal silicon dioxide and 0.6 to 0.8% by weight of magnesium stearate, characterized in that 72.4 to 78.8% by weight of 1-cyclopropyl-6-fluoro-1,4-dihydro-4-oxo-7-(1-piperazinyl)-quinoline-3-carboxylic acid hydrochloride monohydrate are mixed with 7.0 to 9.0% by weight of monocrystalline cellulose, 9.0 to 12% by weight of maize starch and 0.6 to 0.8% by weight of colloidal silicon dioxide, water is added to the mixture until a composition which can be granulated is formed, the mixture is granulated and the granules are dried and, if appropriate, sieved the pore widths being 0.8 to 2 mm, and then mixed with 4.0 to 5.0% by weight of a disintegration auxiliary based on crosslinked polyvinylpyrrolidone and 0.6 to 0.8% by weight of a lubricant.

3. Process for the preparation of capsules or tablets containing the active compound 1-cyclopropyl-6-fluoro-1,4-dihydro-4-oxo-7-(1-piperazinyl)-quinoline-3-carboxylic acid, characterized in that formulations obtained according to Claims 1 and 2 are introduced into capsules or pressed to tablets.

**Revendications**

**Revendications pour les Etats Contractants : AT, BE, CH, DE, FR, GB, IT, LI, NL, SE**

1. Préparations pharmaceutiques, contenant
   72,4 à 78,8% en poids d'acide 1-cyclopropyl-6-fluoro-1,4-dihydro-4-oxo-7-(1-pipérazinyl)-quinoléine-3-carboxylique sous la forme du chlorhydrate monohydraté,
   7,0 à 9,0% en poids d'un liant desséchant à base de cellulose,
   9,0 à 12,0% en poids d'un adjuvant de désintégration à base d'amidon,
   4,0 à 5,0% en poids d'un adjuvant de désintégration à base de dérivés cellulosiques et/ou de polyvinylpyrrolidones réticulées,
   0,6 à 0,8% en poids d'un agent améliorant l'écoulement et
   0,6 à 0,8% en poids d'un lubrifiant.

2. Préparations pharmaceutiques suivant la revendication 1, caractérisées en ce qu'elles contiennent
   72,4 à 78,8% en poids d'acide 1-cyclopropyl-6-fluoro-1,4-dihydro-4-oxo-7-(1-pipérazinyl)-quinoléine-3-carboxylique sous la forme du chlorhydrate monohydraté,
   7,0 à 9,0% en poids de cellulose microcristalline,
   9,0 à 12,0% en poids d'amidon de maïs,
   4,0 à 5,0% en poids de polyvinylpyrrolidone réticulée,
   0,6 à 0,8% en poids de silice colloïdale,
   0,6 à 0,8% en poids de stéarate de magnésium.

3. Procédé pour obtenir des préparations pharmaceutiques suivant la revendication 1, caractérisé en ce qu'on mélange
   72,4 à 78,8% en poids de monohydrate de chlorhydrate de 1-cyclopropyl-6-fluoro-1,4-dihydro-4-oxo-7-(1-pipérazinyl)-quinoléine-3-carboxylique,
   7,0 à 9,0% en poids d'un liant desséchant à base de cellulose,
   9,0 à 12,0% en poids d'un adjuvant de désintégration à base d'amidon et
   0,6 à 0,8% en poids d'un agent améliorant l'écoulement, on ajoute de l'eau au mélange jusqu'à formation d'une masse apte à la granulation, on granule la masse, on sèche les granulés, on les tamise le cas échéant, les ouvertures de mailles allant de 0,8 à 2 mm, puis on les mélange avec 4,0 à 5,0% en poids d'un adjuvant de désintégration à base de dérivés cellulosiques et/ou de polyvinylpyrrolidones réticulées et
   0,6 à 0,8% en poids d'un lubrifiant.

4. Procédé de production de gélules ou de comprimés qui contiennent l'acide 1-cyclopropyl-6-fluoro-1,4-dihydro-4-oxo-7-(1-pipérazinyl)-quinoléine-3-carboxylique comme substance active, caractérisé en ce qu'on charge des préparations suivant les revendications 1 et 2 dans des gélules ou on les presse en comprimés.

5. Gélules contenant des préparations pharmaceutiques suivant les revendications 1 et 2.

6. Comprimés constitués de préparations pharmaceutiques suivant les revendications 1 et 2.

**Revendications pour les Etats Contractants : ES, GR**

1. Procédé de production de préparations pharmaceutiques, contenant 72,4 à 78,8% en poids d'acide 1-cyclopropyl-6-fluoro-1,4-dihydro-4-oxo-7-(1-pipérazinyl)-quinoléine-3-carboxylique sous la forme du chlorhydrate monohydraté,
   7,0 à 9,0% en poids d'un liant desséchant à base de cellulose,
   9,0 à 12,0% en poids d'un adjuvant de désintégration à base d'amidon,
   4,0 à 5,0% en poids d'un adjuvant de désintégration à base de dérivés cellulosiques et/ou de polyvinylpyrrolidones réticulées,
   0,6 à 0,8% en poids d'un agent améliorant l'écoulement et
   0,6 à 0,8% en poids d'un lubrifiant, caractérisé en ce qu'on mélange 72,4 à 78,8% en poids de chlorhydrate monohydraté d'acide 1-cyclopropyl-6-fluoro-1,4-dihydro-4-oxo-7-(1-pipérazinyl)-quinoléine-3-carboxylique avec 7,0 à 9,0% en poids d'un liant desséchant à base de cellulose, 9,0 à 12,0% en poids d'un adjuvant de désintégration à base d'amidon et 0,6 à 0,8% en poids d'un agent améliorant l'écoulement, on ajoute de l'eau au mélange jusqu'à formation d'une masse apte à la granulation, on granule la masse, on sèche les granulés, on les tamise le cas échéant, les ouvertures de mailles allant de 0,8 à 2 mm, puis on les mélange avec 4,0 à 5,0% en poids d'un adjuvant de désintégration à base de dérivés cellulosiques et/ou de polyvinylpyrrolidones réticulées, et 0,6 à 0,8% en poids d'un lubrifiant.

2. Procédé pour l'obtention de préparations pharmaceutiques suivant la revendication 1, contenant
   72,4 à 78,8% en poids d'acide 1-cyclopropyl-6-fluoro-1,4-dihydro-4-oxo-7-(1-pipérazinyl)-quinoléine-3-carboxylique sous la forme du chlorhydrate monohydraté,

7,0 à 9,0% en poids de cellulose microcristalline,

9,0 à 12,0% en poids d'amidon de maïs,

4,0 à 5,0% en poids de polyvinylpyrrolidone réticulée,

0,6 à 0,8% en poids de silice colloïdale,

0,6 à 0,8% en poids de stéarate de magnésium, caractérisé en ce qu'on mélange 72,4 à 78,8% en poids de chlorhydrate monohydraté d'acide 1-cyclopropyl-6-fluoro-1,4-dihydro-4-oxo-7-(1-pipérazinyl)-quinoléine-3-carboxylique avec 7,0 à 9,0% en poids de cellulose microcristalline, 9,0 à 12,0% en poids d'amidon de maïs et 0,6 à 0,8% en poids de silice colloïdale, on ajoute de l'eau au mélange jusqu'à l'obtention d'une masse apte à la granulation, on granule la masse, on sèche les granulés, on les tamise le cas échéant, les ouvertures de mailles allant de 0,8 à 2 mm, puis on les mélange avec 4,0 à 5,0% en poids d'un adjuvant de désintégration à base de polyvinylpyrrolidone réticulée et 0,6 à 0,8% en poids d'un lubrifiant.

3. Procédé de préparation de gélules ou de comprimés, qui contiennent comme substance active l'acide 1-cyclopropyl-6-fluoro-1,4-dihydro-4-oxo-7-(1-pipérazinyl)-quinoléine-3-carboxylique, caractérisé en ce que les préparations obtenues selon les revendications 1 et 2 sont chargées dans des capsules ou pressées en comprimés.